# NEUE EUROPÄISCHE PATENTSCHRIFT

(11) **EP 3 694 353 B2**
(45) Veröffentlichungstag und Bekanntmachung des Hinweises auf die Entscheidung über den Einspruch: **08.10.2025**
(45) Hinweis auf die Patenterteilung: 23.11.2022
(21) Anmeldenummer: 18786737.9
(22) Anmeldetag: 11.10.2018
(51) Int. Cl.: A61M 15/06, A61M 11/04, A61M 15/02, A24F 40/44, A24F 40/46

(54) **VERDAMPFEREINHEIT FÜR EINEN INHALATOR, INSBESONDERE FÜR EIN ELEKTRONISCHES ZIGARETTENPRODUKT**
EVAPORATOR UNIT FOR AN INHALER, IN PARTICULAR FOR AN ELECTRONIC CIGARETTE PRODUCT
CARTOUCHE DE LIQUIDE DESTINÉE À UN INHALATEUR, NOTAMMENT À UN PRODUIT CIGARETTE ÉLECTRONIQUE

(30) Priorität: 13.10.2017 DE 102017123868
(43) Veröffentlichungstag der Anmeldung: 19.08.2020
(73) Patentinhaber: Körber Technologies GmbH, 21033 Hamburg (DE)
(72) Erfinder: TRIEU, Hoc Khiem, 21394 Westergellersen (DE); KALAYDZHYAN, Karen, 22607 Hamburg (DE); BOHNE, Sven, 22297 Hamburg (DE)
(74) Vertreter: Müller Verweyen
(86) Internationale Anmeldenummer: PCT/EP2018/077737
(87) Internationale Veröffentlichungsnummer: WO 2019/072969

(56) Entgegenhaltungen:
- EP-A1- 3 117 860
- EP-A1- 3 372 096
- WO-A1-2017/139963
- WO-A1-2018/083007
- WO-A1-2018/083007
- WO-A1-2018/211035
- WO-A1-2019/052748
- US-A1- 2006 196 968
- US-A1- 2009 220 222
- US-A1- 2009 224 064
- US-A1- 2015 059 780
- US-A1- 2016 345 630

## Beschreibung

Die vorliegende Erfindung betrifft eine Verdampfereinheit für einen Inhalator, insbesondere für ein elektronisches Zigarettenprodukt, mit einem elektrisch betreibbaren, insbesondere planaren Heizkörper, der eine Einlassseite und eine Auslassseite aufweist, und einer Mehrzahl von Mikrokanälen, die sich jeweils von der Einlassseite zu der Auslassseite durch den Heizkörper erstrecken, wobei der Heizkörper durch Anlegen einer Heizspannung zum Verdampfen von durch die Mikrokanäle geförderter Flüssigkeit eingerichtet ist.

Im Stand der Technik erfolgt die Liquidzufuhr zum Heizkörper typischerweise kapillar mittels eines Dochts. Die verwendeten Dochte haben entlang der Förderrichtung idealerweise eine konstante Förderwirkung. Ist die Förderrate geringer als die geforderte Verdampfungsrate, so trocknet der Docht in direkter Nähe zum Heizkörper aus. Es folgt ein Trockenzug (sogenannter Dry Puff) und Schadstoffe werden freigesetzt.

Im Falle eines planaren Heizkörpers muss der Heizkörper zu jeder Zeit und an jedem Ort durch den Docht möglichst gleichmäßig benetzt werden, um eine konstante Temperaturverteilung und damit gleichmäßige, schadstofffreie Verdampfung über seine Oberfläche zu gewährleisten.

Im aktuellen Stand der Technik werden Heizer für E-Zigaretten auf Si-Basis vorgeschlagen. Keines dieser Systeme ist bisher umgesetzt worden. Eine besondere Schwierigkeit ergibt sich in der elektrischen, mechanischen und hydraulischen Ankopplung der Heizer bei gleichzeitiger thermischer Entkopplung der Struktur und der Vermeidung von Übergangsverlusten.

Die US 2015/0059780 A1 offenbart einen elektronischen Rauchartikel, umfassend einen Zerstäube aus einem elektrisch leitenden, porösen Kohlenstoffheizer und einem Transportelement.

Aus der EP 3 117 860 A1 ist eine Inhalatorkomponente für die intermittierende, inhalations- oder zugsynchrone Bildung eines Dampf-Luft-Gemisches oder/und Kondensationsaerosols bekannt, umfassend ein Verbund bestehend aus einem Docht mit einer Kapillarstruktur und einem Heizelement.

Die WO 2018/083007 A1 offenbart einen Inhalator, umfassend einen Verdampfungskörper aus einem elektrisch leitfähigen Substrat, einer Mehrzahl von Mikrokanälen und einem Widerstandsheizelement.

Die Aufgabe der Erfindung besteht darin, eine Verdampfereinheit bereitzustellen, bei der Probleme infolge von Blasenbildung und Trockenlaufen an der Einlassseite des Heizkörpers vermieden werden.

Die Erfindung löst diese Aufgabe mit den Merkmalen der unabhängigen Ansprüche.

Mittels der erfindungsgemäßen Dochtstruktur kann der Blasenbildung im Einlassbereich des Heizkörpers entgegenwirkt werden. Bläschen, die in den Mikrokanälen des Heizkörpers entstehen, können nicht in den Bereich stromaufwärts von der Einlassseite vordringen und zu einem Trockenlaufen des Einlassbereichs des Heizkörpers, und somit zu einer Funktionsbeeinträchtigung des Verdampfers führen. Etwaige Bläschen im Bereich der Dochtstruktur sind in deren Poren bzw. Kapillaren gefangen und können sich nicht großen Blasen vereinigen. Wichtig ist dabei, dass die Dochtstruktur flächig und kontaktierend an der Einlassseite an dem Heizkörper anliegt und sämtliche Mikrokanäle an der Einlassseite überdeckt, damit einzelne Bläschen, die in den Mikrokanälen entstehen, nicht in der falschen Richtung, nämlich auf der Einlassseite zum Flüssigkeitsspeicher hin, aus den Mikrokanälen austreten können. Vielmehr sorgt die Blockade der Einlassseite durch die erfindungsgemäße Dochtstruktur dafür, dass in den Mikrokanälen entstehende Bläschen in den Mikrokanälen zur Auslassseite hin wandern, wo sie aus den Mikrokanälen ausgetrieben werden und dann keine Probleme mehr bereiten können.

Damit der Teil der Dochtstruktur an der Einlassseite des Heizkörpers gleichmäßig mit Liquid versorgt wird, ist es vorteilhaft, das Liquid dort langsamer und gleichmäßiger zu transportieren, als in dem vom Heizkörper weiter entfernten und näher am Liquidreservoir befindlichen Bereich der Dochtstruktur. Konsequenterweise besitzen die Dochtbereiche vorteilhafterweise einen Gradienten der Poren-/Kapillargröße von groß zu klein in Richtung zum Heizkörper. Dies hat zur Folge, dass die Förderrate längs der Dochtstruktur in Richtung zum Heizkörper hin vorteilhaft abnimmt. Die Förderrate der Dochtstruktur hängt bei konstanter Oberflächenspannung und Viskosität des Liquids einzig von der Hydrophobizität und der Poren-/Kapillargröße des Dochtmaterials ab.

Die mittlere Größe der Poren oder Kapillaren in dem porösen bzw. kapillaren Material der Dochtstruktur unterliegt vorteilhaft bestimmten Vorgaben. So ist die mittlere Poren-/Kapillargröße von Poren oder Kapillaren in der Dochtstruktur im Kontaktbereich zu dem Heizkörper vorteilhaft minimal, und besonders vorteilhaft kleiner, vorzugsweise um mindestens eine Größenordnung kleiner als der kleinste Abstand zweier Mikrokanäle zueinander. Auf diese Weise wird vorteilhaft verhindert, dass in einem Mikrokanal entstehende Blasen an der Einlassseite des Heizkörpers in einen benachbarten Mikrokanal übertreten bzw. durchschlagen können. Mit anderen Worten wird durch das beschriebene Merkmal verhindert, dass einzelne Mikrokanäle über das Liquid im Einlassbereich miteinander kommunizieren können. Andernfalls könnte sich durch wechselwirkende Mikrokanäle kein stabiler Verdampfungszustand einzelner Mikrokanäle einstellen. Außerdem können in der Dochtstruktur an der Einlassseite des Heizkörpers entstehende große Dampfblasen mehrere Mikrokanäle an ihren Einlassöffnungen gleichzeitig blockieren, wodurch diese überhitzen könnten.

Erfindungsgemäß ist die mittlere Poren-/Kapillargröße von Poren oder Kapillaren in der Dochtstruktur im Kontaktbereich zu dem Heizkörper kleiner, vorzugsweise um mindestens eine Größenordnung kleiner als der kleinste lichte (bzw. hydraulische) Durchmesser der Mikrokanäle. Auf diese Weise wird wirksam verhindert, dass in den Mikrokanälen entstehende Bläschen durch die Poren oder Kapillaren unerwünscht in das Innere der Dochtstruktur vordringen können. Des Weiteren wird im Inneren der Dochtstruktur die maximal mögliche Blasengröße vorteilhaft auf die Poren- bzw. Kapillargröße beschränkt.

In einer besonders vorteilhaften Ausführungsform ist die mittlere Poren-/Kapillargröße über die Dochtstruktur nicht konstant, sondern verändert sich, insbesondere verringert sich (vorzugsweise monoton) mit abnehmendem Abstand zu dem Heizkörper. Eine monotone Abnahme der Poren-/Kapillargröße schließt dabei generell ein, dass die Poren-/Kapillargröße abschnittweise gleich bleibt, aber jedenfalls mit abnehmendem Abstand zum Heizkörper nicht zunimmt.

Der Liquidtransport von der Dochtstruktur in die Mikrokanäle geschieht durch die Erwärmung des Liquids unterhalb des Heizkörpers, die damit verbundene Absenkung der Oberflächenspannung des Liquids und weiterhin durch die somit verstärkten Kapillarkräfte. Das im Heizkörper verdampfende Liquid führt zu einer Druckdifferenz sowie einer Konzentrationsänderung mit Gefälle in Richtung der Austrittsseite des Heizkörpers. Die Druckdifferenz verstärkt den Kapillardruck innerhalb der einzelnen Mikrokanäle und führt gleichermaßen dazu, dass das verdampfende Liquid einzig von der Dochtstruktur weg verdampfen kann, ohne benachbarte Mikrokanäle zu beeinflussen.

Mittlere Poren-/Kapillargröße bedeutet hier gemittelt über eine Mehrzahl von entsprechenden Poren oder Kapillaren. Mittlere Porengröße bedeutet Mittelwert über den Durchmesser von Kugeln, die jeweils das gleiche Volumen wie die entsprechenden Poren haben. Mittlere Kapillargröße bedeutet Mittelwert über den Durchmesser von Zylindern, die jeweils die gleiche Länge und das gleiche Volumen wie die entsprechenden Kapillaren haben.

Die zuvor beschriebene Dochtstruktur kann auf mehrere Arten vorteilhaft umgesetzt werden. In einer vorteilhaften Ausführungsform kann die Dochtstruktur diskret oder mehrstufig aufgebaut sein und eine Mehrzahl von aneinander anliegenden porösen und/oder kapillaren Schichten mit einer jeweils eine konstanten mittlere Poren-/Kapillargröße aufweisen. Diese Ausführungsform kann fertigungstechnisch besonders einfach herstellbar sein.

In einer alternativen Ausführungsform kann die Dochtstruktur als ein Element mit gradueller Veränderung der mittleren Poren-/Kapillargröße in Abhängigkeit vom Abstand zu dem Heizkörper aufgebaut sein. In dieser Variante liegt daher nur eine Schicht mit gradueller und monotoner Veränderung, insbesondere Abnahme der Poren-/Kapillargröße in Richtung zu dem Heizkörper hin vor. Dies hat den Vorteil einer verringerten Teilezahl.

Mischformen der zuvor beschriebenen Ausführungsformen sind möglich. Beispielsweise kann im Bereich der Einlassseite des Heizkörpers eine Schicht mit konstanter, geringer mittlerer Poren-/Kapillargröße vorgesehen sein und daran anliegend, zum Flüssigkeitsspeicher hin, eine Schicht mit stetig bzw. kontinuierlich zunehmender mittlere Poren-/Kapillargröße.

Vorteilhaft ist die Förderrate der Dochtstruktur mindestens so groß ist wie die maximale Verdampfungsrate des Heizkörpers. Damit wird jederzeit eine ausreichende Flüssigkeitsnachführung sichergestellt, so dass ein nachteiliges Leerlaufen des Heizkörpers verhindert wird. Die Verdampfungsrate wird dabei bestimmt durch die Geometrie der Heizkörperstruktur (Volumen vs. Oberfläche) und die Verdampferleistung.

Demnach ist die kapillare Dochtstruktur eingerichtet, um das Liquid gleichmäßig über ihr gesamtes Volumen zum Heizkörper fördern. Förderrate der Dochtstruktur und Verdampfungsrate des Heizkörpers sind so zueinander eingestellt, dass die Förderrate über den gesamten Betriebsbereich mindestens die Verdampfungsrate bedienen kann. Auf diese Weise wird verhindert, dass während des Verdampfungsvorgangs zu wenig Liquid am Heizkörper anliegt, wodurch dieser austrocknen würde.

Vorteilhaft kann die Dochtstruktur ganz oder teilweise bestehen aus Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, und/oder einem Verbund von zwei oder mehr der vorgenannter Materialien.

Vorzugsweise sind Frequenz und/oder Tastgrad einer Heizspannung Uh für den Heizkörper an die Eigenschwingung und/oder Eigenfrequenz von Schwingungen von in den Mikrokanälen entstehenden Gasblasen angepasst. Dem liegt die Erkenntnis zugrunde, dass einer Verdampfung mit passiver kapillaren Liquidzufuhr, wie im Falle der Erfindung mittels der Dochtstruktur, anderen Gesetzmäßigkeiten unterliegt als eine Verdampfung mit aktiver Zuführung des Liquids. Zur Optimierung der Verdampfung im Sinne einer gleichmäßigen, schadstofffreien Dampferzeugung, welche unter anderem abhängig ist von der Förderrate des Liquids zu dem Heizkörper, wird die Heizspannung für den Heizkörper vorteilhaft derart gepulst angesteuert, dass die Förderrate an die Eigenschwingung der Blasenentstehung während des Blasensiedens in den Mikrokanälen angepasst ist.

Vorzugsweise wird dabei die Frequenz und/oder der Tastgrad der Heizspannung Uh je nach Mischungsverhältnis der zu verdampfenden Flüssigkeit optimal gewählt, da die Eigenfrequenzen der Blasenschwingungen der in Frage kommenden Flüssigkeiten und -gemische variieren können.

Es hat sich aber gezeigt, dass eine bevorzugte Heizfrequenz im Bereich zwischen 20 Hz und 200 Hz, vorzugsweise im Bereich zwischen 25 Hz und 100 Hz, noch weiter vorzugsweise im Bereich zwischen 30 Hz und 70 Hz und beispielsweise 50 Hz einen großen Teil der in Frage kommenden Flüssigkeiten und -gemische abdeckt.

Ebenfalls bevorzugt beträgt der durch die Heizspannung Uh für den Heizkörper erzeugte maximale Heizstrom nicht mehr als 7 A, um konzentrierten Dampf bei Vermeidung von Überhitzung zu gewährleisten.

Vorzugsweise ist mindestens ein Vorspannungs-erzeugendes Klemmelement vorgesehen, das zur Klemmung des Heizkörpers auf den Träger angeordnet und eingerichtet ist. Mittels des Klemmelements wird der Heizkörper auf dem Träger festgeklemmt und auf diese Weise sicher und unverrückbar in der Verdampfereinheit gehalten.

In einer besonders bevorzugten Ausführungsform dient das mindestens eine Klemmelement gleichzeitig als Elektrode zur elektrischen Kontaktierung und Versorgung des Heizkörpers. In diesem Fall sind separate Elektroden für die elektrische Kontaktierung des Heizkörpers entbehrlich.

Vorzugsweise sind mindestens zwei Klemmelemente auf gegenüberliegenden Seiten des Heizkörpers vorgesehen, was eine besonders hohe mechanische Stabilität mit relativ geringem Aufwand ermöglicht. In einer bevorzugten Ausführungsform weist das mindestens eine Klemmelement einen den Heizkörper linienförmig kontaktierenden Klemmbügel auf. Aufgrund der Linienkontaktierung zwischen dem Klemmbügel und dem Heizkörper ergibt sich eine ausgezeichnete elektrische Verbindung zwischen dem Klemmelement und dem Heizkörper, bei gleichzeitig idealer thermischer Entkopplung zwischen dem Klemmelement und dem Heizkörper wegen fehlendem Flächenkontakt.

Das Klemmelement kann den Heizkörper seitlich parallel zur Auslassseite und/oder senkrecht auf die Auslassseite und/oder in einer Ecknut des Trägers klemmen. Die letztgenannte Möglichkeit involviert zwei Kontaktlinien zwischen dem Klemmbügel und dem Heizkörper, was die elektrische Kontaktierung weiter erheblich verbessert. Ein Klemmelement kann auch mehr als einen Klemmbügel aufweisen, insbesondere beliebige zwei oder alle drei Klemmbügel der vorgenannten Art.

In einer vorteilhaften Ausführungsform kann mindestens ein sich durch eine Bohrung des Trägers erstreckender elektrischer Leiter zur elektrischen Kontaktierung des Klemmelements vorgesehen sein, der insbesondere eine Leiterplatte kontaktiert, die auf der von dem Heizkörper abgewandten Seite des Trägers beabstandet angeordnet ist. Es ist aber auch vorteilhaft möglich, dass der Träger selbst als Leiterplatte ausgebildet ist, was die Anzahl der Teile und somit den Herstellungsaufwand reduziert.

Es wird demnach eine vorteilhafte Verdampfereinheit vorgeschlagen, in der ein insbesondere planarer Heizkörper beispielsweise auf zwei gegenüberliegenden Seiten linienförmig geklemmt und dadurch gleichzeitig elektrisch kontaktiert wird. Durch die federnde Klemmung wird ein Anpressdruck auf das unter dem Heizer liegende Dochtmaterial ausgeübt, welche den Verdampfungsdruck ausgleicht. Die Dochtstruktur ist in einer Durchgangsöffnung eines Trägers gehalten und unter dem Träger ist das Liquidreservoir angebunden. Die Klemmung wird so vorgenommen, dass Lufteinschlüsse zwischen Heizkörper und Docht vermieden werden.

Die insbesondere planare, mechanisch stabile Struktur des Heizkörpers begünstigt die gleichmäßige, flächige Anpressung an die darunter liegende Dochtstruktur und somit eine gleichmäßige Liquidversorgung. Die Dochtstruktur endet vorteilhaft erhaben oberhalb des Trägers. Dadurch hat der aufliegende Heizkörper keinen weiteren mechanischen Kontakt zur Umgebung und es entstehen keine Wärmebrücken.

Der elektrische Kontakt ist so angelegt, dass bei minimal möglicher Kontaktfläche der maximal benötigte Heizstrom übertragen werden kann. Der linienförmige Kontakt ist gleichmäßig über die jeweiligen Seiten des Heizkörpers ausgebildet, so dass keine unnötigen Übergangsverluste durch ungenaue Kontaktierung entstehen, welche zu einer unregelmäßigen Erwärmung in den Randbereichen des Heizkörpers führen. Die Kontaktierung Metall-Silizium vermeidet auch Dioden-Effekte nach Schottky durch Ausbildung eines Eutektikums, so dass der Ladungsträgertransport vorteilhaft dem Ohm'schen Gesetz folgt.

Die Erfindung wird im Folgenden anhand bevorzugter Ausführungsformen unter Bezugnahme auf die beigefügten Figuren erläutert. Dabei zeigt
- Fig. 1: eine schematische Darstellung eines elektronischen Zigarettenprodu kts;
- Fig. 2: eine schematische Querschnittsansicht einer erfindungsgemäßen Verdampfereinheit;
- Fig. 3: eine perspektivische Querschnittsansicht einer erfindungsgemäßen Verdampfereinheit;
- Fig. 4: eine vergrößerte Darstellung der Verdampfereinheit aus Figur 2 im Auszug;
- Fig. 5, 6: Diagramme zur Illustrierung des funktionalen Zusammenhangs zwischen der mittleren Poren-/Kapillargröße der Dochtstruktur über dem Abstand zum Heizkörper für zwei unterschiedliche Ausführungsformen;
- Fig. 7: eine schematische Querschnittsansicht einer Verdampfereinheit in einer Ausführungsform der Erfindung;
- Fig. 8, 9: Aufsicht auf den Träger einer Verdampfereinheit von der Seite des Heizkörpers (Figur 4) und von der entgegengesetzten Seite der Flüssigkeitszuführung (Figur 5); und
- Fig. 10: eine Querschnittsansicht einer Verdampfereinheit in weiteren Ausführungsformen der Erfindung.

Der Inhalator 10, hier ein elektronisches Zigarettenprodukt, umfasst ein Gehäuse 11, in dem ein Luftkanal 30 zwischen mindestens einer Lufteinlassöffnung 31 und einer Luftauslassöffnung 24 an einem Mundende 32 des Zigarettenprodukts 10 vorgesehen ist. Das Mundende 32 des Zigarettenprodukts 10 bezeichnet dabei das Ende, an dem der Konsument zwecks Inhalation zieht und dadurch das Zigarettenprodukt 10 mit einem Unterdruck beaufschlagt und eine Luftströmung 34 in dem Luftkanal 30 erzeugt.

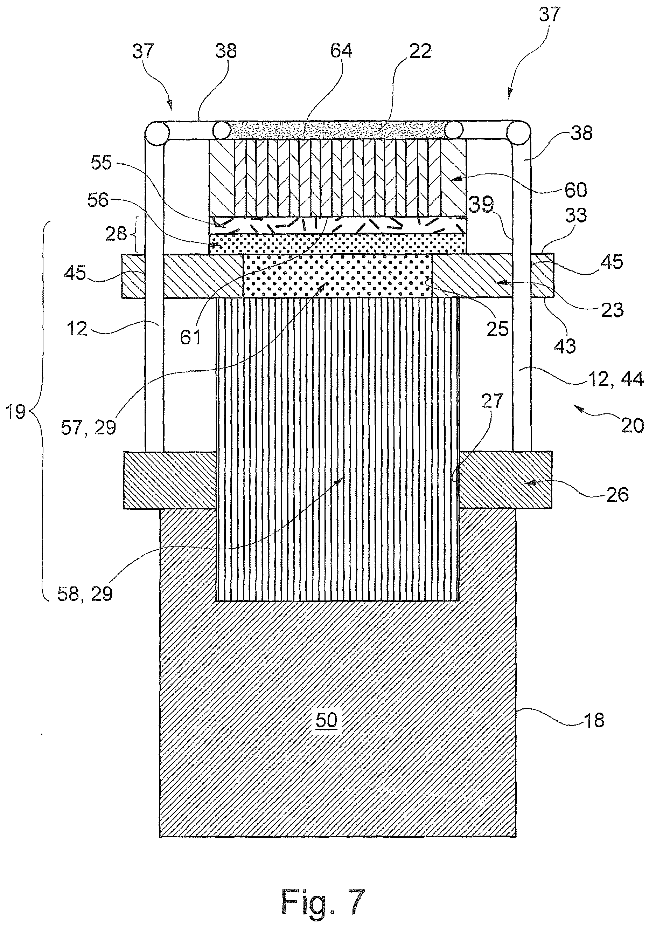

Das Zigarettenprodukt 10 besteht vorteilhaft aus einem Basisteil 16 und einer Verbrauchseinheit 17, die die Verdampfereinheit 20 und den Flüssigkeitsspeicher 18 umfasst und insbesondere in Form einer auswechselbaren Kartusche ausgebildet ist. Die durch die Einlassöffnung 31 angesaugte Luft wird in dem Luftkanal 30 zu oder entlang mindestens einer Verdampfereinheit 20 geleitet. Die Verdampfereinheit 20 ist mit mindestens einem Flüssigkeitsspeicher 18 verbunden oder verbindbar, in dem mindestens eine Flüssigkeit 50 gespeichert ist. Die Verdampfereinheit 20 verdampft Flüssigkeit 50, die ihr aus dem Flüssigkeitsspeicher 18 zugeführt wird, und gibt die verdampfte Flüssigkeit als Aerosol/Dampf 22 (siehe Figur 7) an einer Auslassseite 64 in den Luftstrom 34 zu. Ein vorteilhaftes Volumen des Flüssigkeitsspeichers 18 liegt im Bereich zwischen 0,1 ml und 5 ml, vorzugsweise zwischen 0,5 ml und 3 ml, weiter vorzugsweise zwischen 0,7 ml und 2 ml oder 1,5 ml.

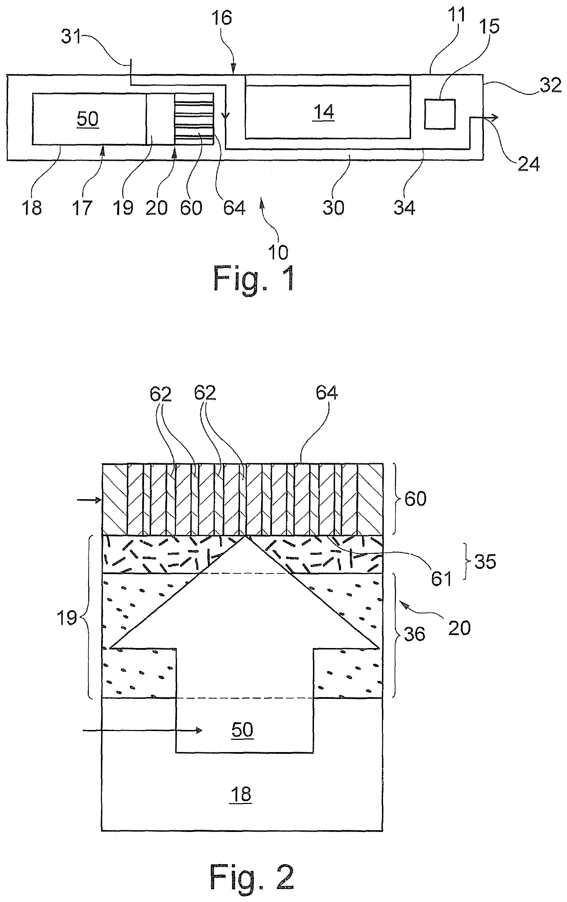

Die elektronische Zigarette 10 umfasst des Weiteren einen elektrischen Energiespeicher 14 und eine elektronische Steuerungsvorrichtung 15. Der Energiespeicher 14 ist in der Regel in dem Basisteil 16 angeordnet und kann insbesondere eine elektrochemische Einweg-Batterie oder ein wiederaufladbarer elektrochemischer Akku, beispielsweise ein Lithium-Ionen-Akku, sein. Die elektronische Steuerungsvorrichtung 15 umfasst mindestens eine digitale Datenverarbeitungseinrichtung, insbesondere Mikroprozessor und/oder Microcontroller, in dem Basisteil 16 (wie in Figur 1 gezeigt) und/oder in der Verbrauchseinheit 17.

In dem Gehäuse 11 ist vorteilhaft ein Sensor, beispielsweise ein Drucksensor oder ein Druck- oder Strömungsschalter, angeordnet, wobei die Steuerungsvorrichtung 15 auf der Grundlage eines von dem Sensor ausgegebenen Sensorsignals feststellen kann, dass ein Konsument am Mundende 32 des Zigarettenprodukts 10 zieht, um zu inhalieren. In diesem Fall steuert die Steuerungsvorrichtung 15 die Verdampfereinheit 20 an, um Flüssigkeit 50 aus dem Flüssigkeitsspeicher 18 als Aerosol/Dampf in den Luftstrom 34 zuzugeben.

Die in dem Flüssigkeitsspeicher 18 gespeicherte, zu dosierende Flüssigkeit 50 ist beispielsweise eine Mischung aus 1,2-Propylenglykol, Glycerin, Wasser, mindestens einem Aroma (Flavour) und/oder mindestens einem Wirkstoff insbesondere Nikotin.

Die Verbrauchseinheit bzw. Kartusche 17 umfasst vorteilhaft einen nichtflüchtigen Datenspeicher zum Speichern von die Verbrauchseinheit bzw. Kartusche 17 betreffender Information bzw. Parameter. Der Datenspeicher kann Teil der elektronischen Steuerungsvorrichtung 15 sein. In dem Datenspeicher ist vorteilhaft Information zur Zusammensetzung der in dem Flüssigkeitsspeicher 18 gespeicherten Flüssigkeit, Information zum Prozessprofil, insbesondere Leistungs-/Temperatursteuerung; Daten zur Zustandsüberwachung bzw. Systemprüfung, beispielsweise Dichtigkeitsprüfung; Daten betreffend Kopierschutz und Fälschungssicherheit, eine ID zur eindeutigen Kennzeichnung der Verbrauchseinheit bzw. Kartusche 17, Seriennummer, Herstelldatum und/oder Ablaufdatum, und/oder Zugzahl (Anzahl der Inhalationszüge durch den Konsumenten) bzw. der Nutzungszeit gespeichert. Der Datenspeicher ist vorteilhaft über Kontakte und/oder Leitungen mit der Steuereinrichtung 15 verbunden oder verbindbar.

Eine vorteilhafte Ausführungsform einer erfindungsgemäßen Verdampfereinheit 20 ist in Fig. 2 gezeigt. Die Verdampfereinheit 20 umfasst einen blockförmigen, vorzugsweise monolithischen Heizkörper 60 vorzugsweise aus einem elektrisch leitenden Material, vorzugsweise Silizium, dotierte Keramik, Metall-Keramik, Filter-Keramik, Halbleiter, insbesondere Germanium, Graphit, Halbmetall und/oder Metall. Es ist nicht erforderlich, dass der gesamte Heizkörper 60 aus einem elektrisch leitenden Material besteht. Es kann beispielsweise ausreichen, dass die Oberfläche des Heizkörpers 60 elektrisch leitend, beispielsweise metallisch, beschichtet ist. In diesem Fall muss nicht die gesamte Oberfläche beschichtet sein, beispielsweise können Leiterbahnen auf einem nichtleitenden Grundkörper vorgesehen sein. Es ist auch nicht zwingend erforderlich, dass der gesamte Heizkörper 60 heizt; es kann beispielsweise ausreichen, wenn ein Abschnitt oder eine Heizschicht des Heizkörpers 60 im Bereich der Austrittsseite 64 heizt.

Der Heizkörper 60 ist mit einer Mehrzahl von Mikrokanälen 62 versehen, die eine Einlassseite 61 des Heizkörpers 60 mit einer Auslassseite 64 flüssigkeitsleitend verbinden. Die Einlassseite 61 ist über eine Dochtstruktur 19 flüssigkeitsleitend mit dem Flüssigkeitsspeicher 18 verbunden. Die Dochtstruktur 19 dient zur passiven Förderung von Flüssigkeit aus einem Flüssigkeitsspeicher 50 zu dem Heizkörper 60 mittels Kapillarkräften.

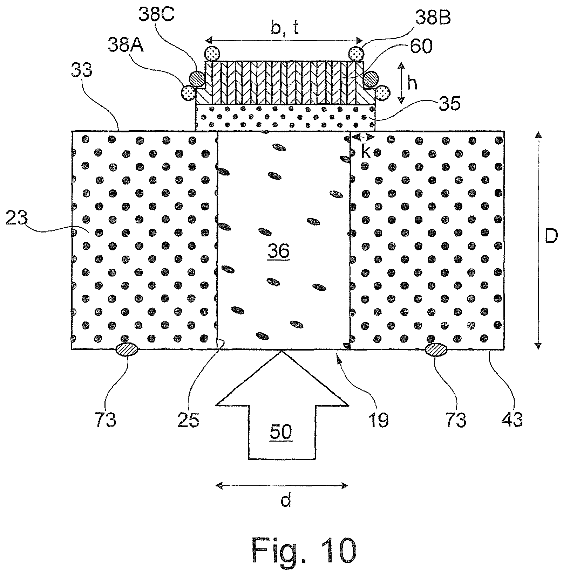

Der mittlere Durchmesser der Mikrokanäle 62 liegt vorzugsweise im Bereich zwischen 5 µm und 200 µm, weiter vorzugsweise im Bereich zwischen 30 µm und 150 µm, noch weiter vorzugsweise im Bereich zwischen 50 µm und 100 µm. Aufgrund dieser Abmessungen wird vorteilhaft eine Kapillarwirkung erzeugt, so dass an der Einlassseite 61 in einen Mikrokanal 62 eindringende Flüssigkeit durch den Mikrokanal 62 nach oben steigt, bis der Mikrokanal 62 mit Flüssigkeit gefüllt ist. Das Volumenverhältnis von Mikrokanälen 62 zu Heizkörper 60, das als Porösität des Heizkörpers 60 bezeichnet werden kann, liegt beispielsweise im Bereich zwischen 10% und 50%, vorteilhaft im Bereich zwischen 15% und 40%, noch weiter vorteilhaft im Bereich zwischen 20% und 30%, und beträgt beispielsweise 25%. Die Kantenlängen der mit Mikrokanälen 62 versehenen Flächen des Heizkörpers 60 liegen beispielsweise im Bereich zwischen 0,5 mm und 3 mm. Die Abmessungen der mit Mikrokanälen 62 versehenen Flächen des Heizkörpers 60 können beispielsweise betragen: 0,95 mm x 1,75 mm oder 1,9 mm x 1,75 mm oder 1,9 mm x 0,75 mm. Die Kantenlängen des Heizkörpers 60 können beispielsweise im Bereich zwischen 0,5 mm und 5 mm liegen, vorzugsweise im Bereich zwischen 0,75 mm und 4 mm, weiter vorzugsweise im Bereich zwischen 1 mm und 3 mm liegen. Die Fläche des Heizkörpers 60 (chip size) kann beispielsweise 1 mm x 3 mm oder 2 mm x 3 mm betragen. Die Breite b des Heizkörpers 60 (siehe Figur 10) liegt vorzugsweise im Bereich zwischen 1 mm und 5 mm, weiter vorzugsweise im Bereich zwischen 2 mm und 4 mm, und beträgt beispielsweise 3 mm. Die Höhe h des Heizkörpers 60 (siehe Figur 10) liegt vorzugsweise im Bereich zwischen 0,05 mm und 1 mm, weiter vorzugsweise im Bereich zwischen 0,1 mm und 0,75 mm, noch weiter vorzugsweise im Bereich zwischen 0,2 mm und 0,5 mm und beträgt beispielsweise 0,3 mm.

Die Anzahl der Mikrokanäle 62 liegt vorzugsweise im Bereich zwischen vier und 1000. Auf diese Weise lässt sich der Wärmeeintrag von dem Substrat in die Mikrokanäle 62 optimieren und eine gesicherte hohe Verdampfungsleistung sowie eine ausreichend große Dampfaustrittsfläche realisieren.

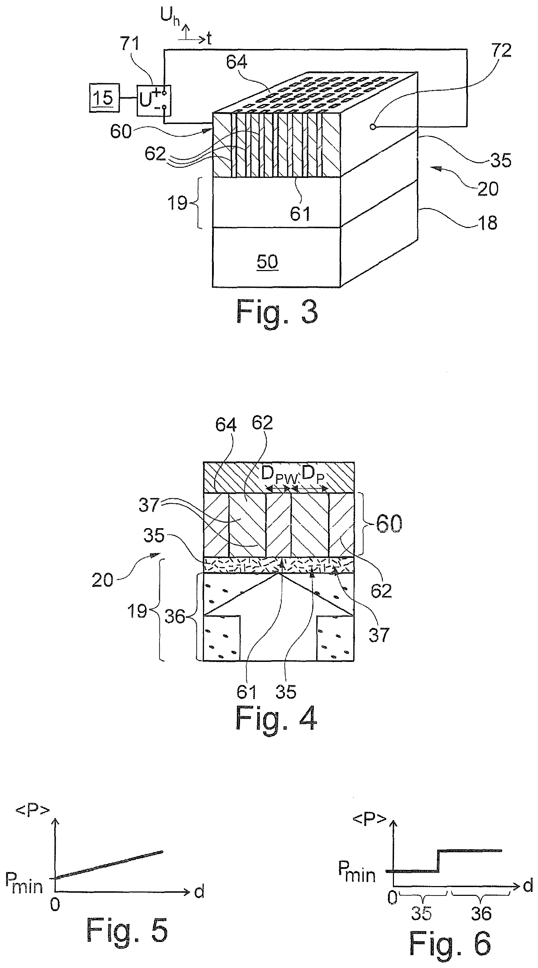

Die Mikrokanäle 62 sind in Form eines quadratischen, rechteckigen, vieleckigen, runden, ovalen oder anders geformten Arrays angeordnet, wie in Figur 3 ersichtlich ist. Das Array kann in Form einer Matrix mit s Spalten und z Zeilen ausgebildet sein, wobei s vorteilhaft im Bereich zwischen 2 und 50 und weiter vorteilhaft im Bereich zwischen 3 und 30 und/oder z vorteilhaft im Bereich zwischen 2 und 50 und weiter vorteilhaft im Bereich zwischen 3 und 30 liegt. Auf diese Weise lässt sich eine effektive und auf einfache Weise herstellbare Anordnung der Mikrokanäle 62 mit gesichert hoher Verdampfungsleistung realisieren.

Der Querschnitt der Mikrokanäle 62 kann quadratisch, rechteckig, vieleckig, rund, oval oder anders geformt sein, und/oder sich in Längsrichtung abschnittweise ändern, insbesondere vergrößern, verkleinern oder konstant bleiben.

Die Länge eines oder jedes Mikrokanals 62 liegt vorzugsweise im Bereich zwischen 100 µm und 1000 µm, weiter vorzugsweise im Bereich zwischen 150 µm und 750 µm, noch weiter vorzugsweise im Bereich zwischen 180 µm und 400 µm und beträgt beispielsweise 300 µm. Auf diese Weise lässt sich eine optimale Flüssigkeitsaufnahme und Portionsbildung bei ausreichend gutem Wärmeeintrag von dem Heizkörper 60 in die Mikrokanäle 62 realisieren.

Der Abstand zweier Mikrokanäle 62 beträgt vorzugsweise mindestens das 1,3-fache des lichten Durchmessers eines Mikrokanals 62, wobei der Abstand auf die Mittelachsen der beiden Mikrokanäle 62 bezogen ist. Der Abstand kann bevorzugt das 1,5- bis 5-fache, weiter bevorzugt das 2- bis 4-fache des lichten Durchmessers eines Mikrokanals 62 betragen. Auf diese Weise lässt sich ein optimaler Wärmeeintrag von dem Substrat in die Mikrokanäle und eine ausreichend stabile Anordnung und Wandstärke der Mikrokanäle realisieren.

Aufgrund der vorbeschriebenen Merkmale kann der Heizkörper 60 auch als Volumenheizer bezeichnet werden.

Die Verdampfereinheit 20 weist eine vorzugsweise von der Steuerungsvorrichtung 15 steuerbare Heizspannungsquelle 71 auf, die über Elektroden 72 an gegenüberliegenden Seiten des Heizkörpers 60 mit diesem verbunden ist, so dass eine von der Heizspannungsquelle 71 erzeugte elektrische Spannung Uh zu einem Stromfluss durch den Heizkörper 60 führt. Aufgrund des Ohmschen Widerstands des elektrisch leitenden Heizkörpers 60 führt der Stromfluss zu einer Erhitzung des Heizkörpers 60 und daher zu einer Verdampfung von in den Mikrokanälen 62 enthaltener Flüssigkeit. Der Heizkörper 60 wirkt somit als Verdampfer. Auf diese Weise erzeugter Dampf/Aerosol entweicht zur Auslassseite 64 aus den Mikrokanälen 62 und wird der Luftströmung 34 beigemischt, siehe Figur 1. Genauer steuert bei Feststellung eines durch Ziehen des Konsumenten verursachten Luftstroms 34 durch den Luftkanal 30 die Steuerungsvorrichtung 15 die Heizspannungsquelle 71 an, wobei durch spontane Erhitzung die in den Mikrokanälen 62 befindliche Flüssigkeit in Form von Dampf/Aerosol aus den Mikrokanälen 62 getrieben wird.

Dabei kann die Dauer der einzelnen Verdampfungsschritte bei unterschiedlichen Temperaturen und/oder einem Verdampfen der einzelnen Komponenten der einzelnen Portionen der Flüssigkeit derart kurz gehalten werden und/oder mit einer Ansteuerfrequenz getaktet erfolgen, dass die schrittweise Verdampfung von einem Konsumenten nicht wahrgenommen und trotzdem eine weitgehend homogene, geschmackskonforme, wiederholbar präzise Aerosolbildung gewährleistet werden kann. Insbesondere erfolgt vorteilhaft zunächst ein Verdampfen einer leichter siedenden Komponente der Flüssigkeit in einem ersten Verdampfungsintervall mit einer ersten Temperatur A und anschließend ein Verdampfen einer höher siedenden Komponente der Flüssigkeit in einem zweiten Verdampfungsintervall mit einer zweiten Temperatur B, welche die Temperatur A übersteigt.

Vorzugsweise ist in dem Datenspeicher des Inhalators 10 eine dem verwendeten Flüssigkeitsgemisch angepasste Spannungskurve Uh(t) hinterlegt. Dies ermöglicht es, den Spannungsverlauf Uh(t) dem verwendeten Liquid angepasst vorzugeben, so dass sich die Heiztemperatur des Heizkörpers 60, und damit auch die Temperatur der kapillaren Mikrokanäle 62, gemäß der bekannten Verdampfungskinetik des jeweiligen Liquids zeitlich über den Verdampfungsvorgang steuern lässt, wodurch optimale Verdampfungsergebnisse erzielbar sind. Die Verdampfungstemperatur liegt vorzugsweise im Bereich zwischen 100°C und 400 °C, weiter bevorzugt zwischen 150°C und 350 °C, noch weiter bevorzugt zwischen 190 C und 290 C.

An der Einlassseite 61 des Heizkörpers 60 ist eine poröse und/oder kapillare, flüssigkeitsleitende Dochtstruktur 19 angeordnet. Die Dochtstruktur 19 kontaktiert die Einlassseite 61 des Heizkörpers 60 flächig und deckt sämtliche Mikrokanäle 62 einlassseitig ab, wie in den Figuren 2 und 3 ersichtlich ist. An der dem Heizkörper 60 gegenüberliegenden Seite ist die Dochtstruktur flüssigkeitsleitend mit dem Flüssigkeitsspeicher verbunden. Die in den Figuren 1 bis 3, 7 gezeigte direkte Anbindung des Flüssigkeitsspeichers 18 an die Dochtstruktur 19 ist nur beispielhaft zu verstehen. Insbesondere können eine Flüssigkeitsschnittstelle und/oder eine mehrere Flüssigkeitsleitungen zwischen Flüssigkeitsspeicher 18 und Dochtstruktur 19 vorgesehen sein. Der Flüssigkeitsspeicher 18 kann daher auch beabstandet von der Dochtstruktur 19 angeordnet sein. Der Flüssigkeitsspeicher 18 kann in seinen Abmessungen größer als die Dochtstruktur 19 sein, siehe beispielsweise Figur 7. Die Dochtstruktur 19 kann beispielsweise in eine Öffnung eines Gehäuses des Flüssigkeitsspeichers 18 eingesetzt sein. Es kann auch eine Mehrzahl von Verdampfereinheiten 20 einem Flüssigkeitsspeicher 18 zugeordnet sein.

Die Dochtstruktur 19 besteht aus porösem und/oder kapillarem Material, das aufgrund von Kapillarkräften in der Lage ist, von dem Heizkörper 60 verdampfte Flüssigkeit in ausreichender Menge von dem Flüssigkeitsspeicher 18 zu dem Heizkörper 60 passiv nachzufördern, um ein Leerlaufen der Mikrokanäle 62 und sich daraus ergebende Probleme zu verhindern.

Die Dochtstruktur 19 besteht vorteilhaft aus einem nichtleitenden Material, um eine unerwünschte Erwärmung von Flüssigkeit in der Dochtstruktur 19 durch Stromfluss zu vermeiden. Die Dochtstruktur 19 besteht vorteilhaft aus einem oder mehreren der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien. In einer vorteilhaften praktischen Ausführungsform kann die Dochtstruktur 19 mindestens ein Keramikfaserpapier und/oder eine poröse Keramik umfassen. Das Volumen der Dochtstruktur 19 liegt vorzugsweise im Bereich zwischen 1 mm³ und 10 mm³, weiter vorzugsweise im Bereich zwischen 2 mm³ und 8 mm³, noch weiter vorzugsweise im Bereich zwischen 3 mm³ und 7 mm³ und beträgt beispielsweise 5 mm³.

Falls die Dochtstruktur 19 aus einem leitenden Material besteht, was nicht ausgeschlossen ist, ist zwischen der Dochtstruktur 19 und dem Heizkörper 60 vorteilhaft eine Isolierschicht aus einem elektrisch und/oder thermisch isolierenden Material, beispielsweise Glas, Keramik oder Kunststoff, mit sich durch die Isolierschicht erstreckenden, mit den Mikrokanälen 62 korrespondierenden Durchgangsöffnungen vorgesehen.

Die Größe der Poren bzw. Kapillaren in dem Material der Dochtstruktur 19 unterliegt bestimmten Vorgaben. So ist die mittlere Poren-/Kapillargröße Dw von Poren oder Kapillaren der Dochtstruktur 19 im Kontaktbereich 35, 61 zu dem Heizkörper 60 vorteilhaft minimal, d.h. Dw = Pmin (siehe Figuren 5, 6), und/oder vorteilhaft kleiner, vorzugsweise um mindestens den Faktor 2, weiter vorzugsweise um mindestens den Faktor 5, als der kleinste Abstand Dp zweier Mikrokanäle 62 zueinander, d.h. Dw « Dp, siehe Figur 4. Des Weiteren ist die mittlere Poren-/Kapillargröße Dw von Poren oder Kapillaren der Dochtstruktur 19 im Kontaktbereich 35, 61 zu dem Heizkörper 60 kleiner, vorzugsweise um mindestens den Faktor 2, weiter vorzugsweise um mindestens den Faktor 5, als der kleinste lichte Durchmesser Dpw eines Mikrokanals 62, d.h. Dw « Dpw.

Die Dochtstruktur 19 im Kontaktbereich 35, 61 zu dem Heizkörper 60 dient dazu, Flüssigkeit gleichmäßig zu verteilen, temperaturbeständig zu sein und mit ihren relativ kleinen Poren und/oder dünnen Kapillaren eine Art Rückschlagventil zu bilden, um unerwünschtes Rückfließen von blasenhaltiger Flüssigkeit aus dem Heizkörper 60 in die Dochtstruktur 19 und/oder in den Flüssigkeitsspeicher 18 zu verhindern.

In der Ausführungsform gemäß Figur 2 weist die Dochtstruktur 19 zwei beispielsweise planare Schichten 35, 36 auf, nämlich eine an der Einlassseite 61 des Heizkörpers 60 flächig anliegende und diese kontaktierende Dochtschicht 35, die als Kontaktschicht bezeichnet werden kann, und eine daran angrenzende Dochtschicht 36, die flüssigkeitsleitend mit dem Flüssigkeitsspeicher 18 verbunden ist und als entferntere Dochtschicht bezeichnet werden kann.

Die Kontaktschicht 35 weist eine in sich im Wesentlichen konstante Poren-/Kapillargrößenverteilung und eine in sich im Wesentlichen konstante mittlere Poren-/Kapillargröße Dw auf, die signifikant kleiner als der kleinste Abstand Dp zweier Mikrokanäle 62 zueinander und signifikant kleiner als der kleinste lichte Durchmesser Dpw eines Mikrokanals 62 ist: Dw « Dp, Dpw.

Die entferntere Dochtschicht 36 weist eine in sich im Wesentlichen konstante Poren-/Kapillargrößenverteilung und eine in sich im Wesentlichen konstante mittlere Poren-/Kapillargröße Dw' auf, die signifikant größer ist als die mittlere Poren-/Kapillargröße Dw der Kontaktschicht 35: Dw' > Dw, aber bevorzugt immer noch kleiner als Dp und/oder Dpw: Dw' < Dp, Dpw.

Der zuvor geschilderte Zusammenhang zwischen der mittlere Poren-/Kapillargröße und dem Abstand d zu dem Heizkörper 60 in der Mehrschichtausführung 35, 36 der Dochtstruktur 19 gemäß Figuren 2 und 4 ist in dem Diagramm gemäß Figur 6 illustriert.

In einer vorteilhaften praktischen Ausführungsform kann die Kontaktschicht 35 beispielsweise eine Faserpapier- oder Keramikpapierschicht und/oder die Schicht 36 eine poröse Keramik sein.

Aus Figur 4 geht ebenfalls hervor, dass in den Mikrokanälen 62 entstehende Luftblasen nicht wesentlich in die Kontaktschicht 35 und somit in die Dochtschicht eindringen und auch nicht in benachbarte Mikrokanäle 62 überspringen können.

Selbstverständlich kann die Dochtstruktur 19 mehr als zwei Dochtschichten 35, 36, ... aufweisen. Auch im Falle von mehr als zwei Dochtschichten 35, 36, ... wird die mittlere Poren-/Kapillargröße mit abnehmendem Abstand zu dem Heizkörper 60 vorteilhaft monoton (d.h. von Dochtschicht zu Dochtschicht) geringer und/oder bleibt gleich, und nimmt daher jedenfalls nicht zu.

In der Ausführungsform gemäß Figur 3 besteht die Dochtstruktur 19 nur aus einer Schicht, deren mittlere Poren-/Kapillargröße mit abnehmendem Abstand d zu dem Heizkörper 60 monoton geringer wird. Der Zusammenhang zwischen mittlerer Poren-/Kapillargröße und dem Abstand d zu dem Heizkörper 60 in der Einschichtausführung gemäß Figur 3 ist in dem Diagramm gemäß Figur 5 illustriert.

In sämtlichen Ausführungsformen kann der gewünschte Poren/Kapillargrößengradient optimal eingestellt und die Flüssigkeitsströmung zum Heizkörper 60 hin verlangsamt und vergleichmäßigt werden.

Die geschilderte Verringerung der mittleren Poren-/Kapillargröße in der Dochtstruktur 19 mit abnehmendem Abstand zum Heizkörper 60 gilt in der Richtung senkrecht zur Einlassseite 61 des Heizkörpers, d.h. senkrecht zur Kontaktfläche zwischen Heizkörper 60 und Dochtstruktur 19, bzw. parallel zum Verlauf der Mikrokanäle 62. Innerhalb einer Sicht mit gleichem Abstand d zu dem Heizkörper 60 ist die mittlere Poren-/Kapillargröße in der Dochtstruktur 19 dagegen vorteilhaft konstant, damit sämtliche Mikrokanäle 62 des Heizkörpers 60 gleichmäßig mit Flüssigkeit versorgt werden.

Die Mikrokanäle 62 sind vorzugsweise mit ihrer Längsachse quer zu den Schichten 19, 35, 36 bzw. allgemeiner zu einer beliebigen Schichtenfolge angeordnet. Auf diese Weise lässt sich ein optimaler Wärmeeintrag von dem Heizkörper 60 in die Mikrokanäle 62 realisieren.

Der Heizkörper 60 kann vorteilhaft aus Teilstücken eines Wafers mit Dünnfilmschichttechnologie hergestellt werden, welcher eine Schichtdicke von vorzugsweise kleiner oder gleich 1000 µm, weiter vorzugsweise 750 µm, noch weiter vorzugsweise kleiner oder gleich 500 µm aufweist. Oberflächen des Heizkörpers 60 können vorteilhaft hydrophil sein. Die Auslassseite 64 des Heizkörpers 60 kann vorteilhaft mikrostrukturiert sein bzw. Mikroausnehmungen (micro grooves) aufweisen.

Die Verdampfereinheit 20 ist so eingestellt, dass eine Flüssigkeitsmenge vorzugsweise im Bereich zwischen 1 µl und 20 µl, weiter vorzugsweise zwischen 2 µl und 10 µl, noch weiter vorzugsweise zwischen 3 µl und 5 µl, typischerweise 4 µl pro Zug des Konsumenten, zudosiert wird. Vorzugsweise kann die Verdampfereinheit 20 hinsichtlich der Flüssigkeits-/Dampfmenge pro Zug, d.h. je Zugdauer von 1 s bis 3 s, einstellbar sein.

Vorteilhafte Ausführungsformen einer Verdampfereinheit 20 sind in den Figuren 7 bis 10 gezeigt.

In dem Ausführungsbeispiel gemäß Figur 7 umfasst die Dochtstruktur 19 mehr als zwei, hier vier Schichten. Unmittelbar angrenzend an den Heizkörper 60, und diesen flächig kontaktierend, ist eine Filterschicht 55 angeordnet, die insbesondere aus einer, zwei oder mehr Mikroglasfaser-Lagen bestehen kann. Daran flächig angrenzend kann eine Faserpapier-Schicht 56 angeordnet sein. Daran flächig angrenzend sind vorteilhaft Dochtschichten 57, 58 vorgesehen, beispielsweise eine Keramikdocht-Schicht 57 und eine Öllampendocht-Schicht 58, d.h. ein Glasfaser-Dochtmaterial, das herkömmlich für die Dochte von Öllampen verwendet wird.

In der Ausführungsform nach Figur 7 erfüllt mindestens die an dem Heizkörper 60 flächig anliegende Schicht 55 vorteilhaft die zuvor erläuterten Bedingungen für die Poren-/Kapillargröße Dw << Dp, Dpw. Vorteilhaft kann auch die Schicht 57 und/oder die Schicht 58 diese Bedingungen erfüllen. Des Weiteren können die Kapillarkräfte für die Kapillarförderung der Flüssigkeit von dem Flüssigkeitsspeicher 18 zu dem Heizkörper 60 überwiegend oder vollständig von den Dochtschichten 57, 58 bereitgestellt werden. Es ist im allgemeinen nicht erforderlich, wenn sämtliche Schichten der Dochtstruktur 19 Kapillarkräfte für die Kapillarförderung der Flüssigkeit bereitstellen. Es kann auch ausreichen, dass nur eine Schicht der Dochtstruktur 19 Kapillarkräfte für die Kapillarförderung der Flüssigkeit bereitstellt.

Die Verdampfereinheit 20 weist einen insbesondere plattenförmigen Träger 23 zum Halten des Heizkörpers 60 und/oder der Dochtstruktur 19 auf, wie in den Figuren 7 bis 10 gezeigt ist. Der Träger 23 kann aus einem geeigneten Material, beispielsweise Keramik, Glas und/oder Kunststoff einschließlich faserverstärktem Kunststoff, beispielsweise Leiterplattenmaterial bestehen und weist eine Durchgangsöffnung 25 auf, durch die sich die Dochtstruktur 19 erstreckt und in der die Dochtstruktur 19 gehalten ist.

Die Dicke D des Trägers 23 (siehe Figur 10) liegt vorzugsweise im Bereich zwischen 0,5 mm bis 4 mm, weiter vorzugsweise im Bereich zwischen 1 mm bis 3 mm, noch weiter vorzugsweise im Bereich zwischen 1 mm und 2 mm und kann beispielsweise 1,6 mm oder 2 mm betragen. Die Dicke einer in der Durchgangsöffnung 25 des Trägers 23 angeordneten Dochtschicht 57 kann an die Dicke des Trägers 23 angepasst sein bzw. dieser entsprechen und daher beispielsweise ebenfalls 1,6 mm oder 2 mm betragen.

Die Durchgangsöffnung 25 ist vorteilhaft kreisrund, was einfach zu fertigen ist. Der Durchmesser d, oder ggf. der mittlere Durchmesser, der Durchgangsöffnung 25 (siehe Figur 10) liegt vorzugsweise im Bereich zwischen 0,5 mm und 4 mm, vorzugsweise im Bereich zwischen 1 mm bis 3 mm, weiter vorzugsweise im Bereich zwischen 1,5 mm und 2,5 mm und beträgt beispielsweise 2 mm.

Der Durchmesser d der Durchgangsöffnung 25 ist kleiner oder gleich, vorteilhaft kleiner als die Breite b des Heizkörpers 60, siehe Figur 10. Das Volumen der Durchgangsöffnung 25, bzw. das Dochtvolumen in der Durchgangsöffnung 25, liegt vorteilhaft im Bereich zwischen 1 mm³ und 8 mm³, vorzugsweise im Bereich zwischen 2 mm³ und 6,5 mm³, weiter vorzugsweise im Bereich zwischen 2,5 mm³ und 5 mm³.

Die Dochtstruktur 19 kann im freien, vormontierten Zustand ein Übermaß, d.h. einen größeren Durchmesser haben als die Durchgangsöffnung 25, um zusätzliche Haltekräfte des Schafts 29 in der Durchgangsöffnung 25 zu erzeugen.

Der Durchmesser t der Dochtschicht 35 (siehe Figur 10) ist vorteilhaft größer als der Durchmesser d der Durchgangsöffnung 25. Vorzugsweise steht die Dochtschicht 35 über ihren gesamten Umfang über die Durchgangsöffnung 25 mit Überstand k über. Aufgrund des allseitigen Überstands der Dochtschicht 35 über die Durchgangsöffnung 25 wird bei einer Klemmung des Heizkörpers 60 auf den Träger 23 die Dochtschicht 35, und somit die gesamte Dochtstruktur 19, sicher in der Verdampfereinheit 20 gehalten.

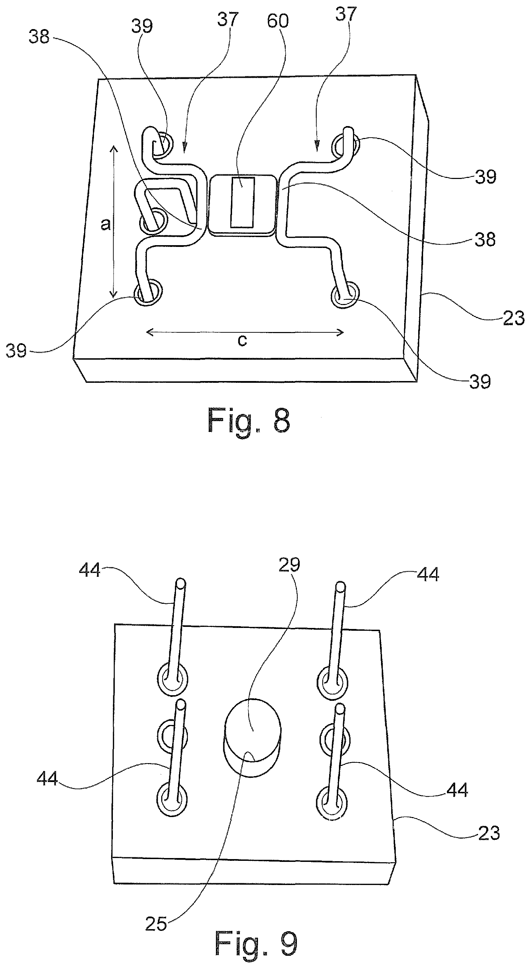

Die Klemmung des Heizkörpers 60 auf dem Träger 23 wird mittels mindestens zwei Klemmelementen 37 bewirkt, siehe insbesondere Figur 8, die an gegenüberliegenden Seiten des Heizkörpers 60 an diesem angreifen. Jedes Klemmelement 37 weist vorteilhaft einen Klemmbügel 38 auf, der an zwei voneinander beabstandeten Befestigungspunkten 39 federnd an dem Träger 23 befestigt ist und eine Vorspannung erzeugt, mittels der der Heizkörper 60 und der Teller 28 auf dem Träger 23 festgeklemmt und somit sicher gehalten wird.

Der Abstand a der beiden Befestigungspunkte 39 eines Klemmbügels 38 voneinander liegt vorzugsweise im Bereich zwischen 4 mm und 10 mm, weiter vorzugsweise im Bereich zwischen 5 mm bis 8 mm und beträgt beispielsweise 6 mm. Der Abstand c zwischen den Befestigungspunkten 39 zweier Klemmbügel 39 voneinander liegt vorzugsweise im Bereich zwischen 5 mm und 12 mm, weiter vorzugsweise im Bereich zwischen 6 mm bis 10 mm und beträgt beispielsweise 8 mm. Die Abmessungen des beispielsweise rechteckigen Trägers 23 liegen vorzugsweise im Bereich zwischen 6 mm und 20 mm, weiter vorzugsweise im Bereich zwischen 8 mm bis 17 mm und noch weiter vorzugsweise im Bereich zwischen 10 mm und 14 mm.

Besonders vorteilhaft dienen die Klemmelemente 37 gleichzeitig als Elektroden zur Kontaktierung des Heizkörpers 60 und dessen Versorgung mit Heizstrom. Zu diesem Zweck bestehen die Klemmelemente 37 bzw. die Klemmbügel 38 vorteilhaft aus einem elektrisch leitenden Material, beispielsweise kann es sich um Metalldraht, beispielsweise Messingdraht handeln. Aufgrund der Linienkontaktierung zwischen dem Klemmbügel 38 und dem Heizkörper 60 ergibt sich eine ausgezeichnete elektrische Verbindung zwischen dem Klemmelement 37 und dem Heizkörper 60, bei gleichzeitig idealer thermischer Entkopplung zwischen dem Klemmelement 37 und dem Heizkörper 60 wegen fehlendem Flächenkontakt. Wärmedissipation von dem Heizkörper 60 in das Klemmelement 37 ist daher gering, die Elektroden 38 bleiben signifikant kühler als der Heizkörper 60. Dioden-Effekte werden vermieden und rein Ohm'scher Ladungsträgertransport ermöglicht.

Der Klemmbügel 38 kann den Heizkörper 60 seitlich parallel zur Auslasseite 64 (Position 38A in Figur 10) und/oder senkrecht auf die Auslassseite 64 (Position 38B in Figur 10) und/oder in einer Ecknut mit einem Zwischenwinkel, beispielsweise zwischen 30° und 60°, sowohl seitlich als auch senkrecht auf die Auslassseite 64 klemmen (Position 38C in Figur 10). Die letztgenannte Möglichkeit involviert zwei Kontaktlinien zwischen dem Klemmbügel 38C und dem Heizkörper 60, was die elektrische Kontaktierung weiter verbessert. Ein Klemmelement 37 kann auch mehr als einen Klemmbügel 38 aufweisen, insbesondere beliebige zwei oder alle drei der Klemmbügel 38A, 38B, 38C.

Die Klemmelemente 37 sind mittels elektrischer Leitungen 12 vorteilhaft mit einer in der Verbrauchseinheit 17 vorgesehenen Leiterplatte 26 (PCB) verbunden, um die elektrische Verbindung zu der elektronischen Steuerungsvorrichtung 15 und zu der Energiequelle 46 für die Stromversorgung des Heizkörpers 60 herzustellen. Auf der Leiterplatte 26 sind vorteilhaft elektronische Komponenten der Verbrauchseinheit 17 angeordnet.

Die Leiterplatte 26 ist in dem Ausführungsbeispiel gemäß Figur 7 ein separates Teil und von dem Träger 23 beabstandet auf dessen dem Heizkörper 60 abgewandter Seite 43 angeordnet. Die Leiterplatte 26 weist eine Durchgangsöffnung 27 auf, durch die sich die Dochtstruktur 19 erstreckt und in der die Dochtstruktur 19 gehalten ist. Die elektrischen Leitungen 12 umfassen hier beispielsweise vier Metallstifte 44, die auf der Seite 33 des Trägers 23 in den Befestigungspunkten 39 mit den Klemmelementen 37 verbunden und jeweils durch eine Durchgangsbohrung 45 durch den Träger 23 hindurchgeführt sind und dann auf der abgewandten Seite 43 den Abstand zwischen dem Träger 23 und der Leiterplatte 26 überbrücken.

In einer anderen Ausführungsform kann der Träger 23 die Leiterplatte 26 ausbilden. Die elektrischen Leitungen 12 können dann entfallen. Es ist auch möglich, dass die Verdampfereinheit 20 selbst keine Leiterplatte umfasst, sondern die Klemmbügel 38 beispielsweise über flexible isolierte Leitungen 12, oder auf andere geeignete Weise, mit einer etwa in dem Basisteil 16 angeordneten Leiterplatte verbunden sind.

An der Unterseite 43 des Trägers 23 kann ein Dichtelement 73, beispielsweise ein Dichtring, zur Abdichtung des Trägers 23 gegen ein Gehäuse des Flüssigkeitsspeichers 18, oder einer anderen unter dem Träger 23 angeordneten Komponente, angeordnet sein, siehe Figur 10.

Im Folgenden wird der Ablauf des Verdampfungsvorgangs erläutert.

In einem Ausgangszustand ist die Spannungsquelle 71 für den Heizvorgang ausgeschaltet.

Zum Verdampfen von Flüssigkeit 50 wird die Spannungsquelle 71 für den Heizkörper 60 aktiviert. Die Spannung Uh wird dabei so eingestellt, dass die Verdampfungstemperatur in dem Heizkörper 60 und somit in den Mikrokanälen 62 an das individuelle Verdampfungsverhalten des eingesetzten Flüssigkeitsgemischs angepasst ist. Dies verhindert die Gefahr von lokaler Überhitzung und dadurch Schadstoffentsteh u ng.

Sobald eine Flüssigkeitsmenge verdampft ist, die dem Volumen der Mikrokanäle 62 entspricht oder damit in Zusammenhang steht, wird die Heizspannungsquelle 71 deaktiviert. Da die Liquideigenschaften und -menge vorteilhaft exakt bekannt sind und der Heizkörper 60 einen messbaren temperaturabhängigen Widerstand aufweist, kann dieser Zeitpunkt sehr genau bestimmt bzw. gesteuert werden. Die Energieaufnahme der Verdampfereinheit 20 lässt sich daher gegenüber bekannten Vorrichtungen reduzieren, da die benötigte Verdampfungsenergie dosierter und damit exakter eingebracht werden kann.

Nach Abschluss des Heizvorgangs sind die Mikrokanäle 62 überwiegend oder vollständig entleert. Die Heizspannung 71 wird dann so lange ausgeschaltet gehalten, bis mittels Nachförderung von Flüssigkeit durch die Dochtstruktur 19 die Mikrokanäle 62 wieder aufgefüllt sind. Sobald dies der Fall ist, kann der nächste Heizzyklus durch einschalten der Heizspannung 71 begonnen werden.

Die von der Heizspannungsquelle 71 erzeugte Ansteuerfrequenz des Heizkörpers 60 liegt im Allgemeinen vorteilhaft im Bereich von 1 Hz bis 50 kHz, bevorzugt im Bereich von 30 Hz bis 30 kHz, noch weiter vorteilhaft im Bereich von 100 Hz bis 25 kHz.

Die Frequenz und der Tastgrad der Heizspannung Uh für den Heizkörper 60 sind vorteilhaft an die Eigenschwingung bzw. Eigenfrequenz der Blasenschwingungen während der Blasensiedung angepasst. Vorteilhaft kann die Periodendauer 1/f der Heizspannung daher im Bereich zwischen 5 ms und 50 ms, weiter vorteilhaft zwischen 10 ms und 40 ms, noch weiter vorteilhaft zwischen 15 ms und 30 ms liegen und beispielsweise 20 ms betragen. Je nach Zusammensetzung der verdampften Flüssigkeit können andere als die genannten Frequenzen optimal an die Eigenschwingung bzw. Eigenfrequenz der Blasenschwingungen angepasst sein.

Des Weiteren hat sich gezeigt, dass der durch die Heizspannung Uh erzeugten maximale Heizstrom vorzugsweise nicht mehr als 7 A, weiter vorzugsweise nicht mehr als 6,5 A, noch weiter vorzugsweise nicht mehr als 6 A betragen und optimalerweise im Bereich zwischen 4 A und 6 A liegen sollten, um konzentrierten Dampf bei Vermeidung von Überhitzung zu gewährleisten.

Die Förderrate der Dochtstruktur 19 ist wiederum optimal an die an die Verdampfungsrate des Heizkörpers 60 angepasst, so dass jederzeit ausreichend Flüssigkeit nachgefördert werden kann und ein Leerlaufen des Bereichs vor dem Heizkörper 60 vermieden wird.

Die Verdampfereinheit 20 ist vorzugsweise auf der Grundlage von MEMS-Technologie, insbesondere aus Silizium, gefertigt und daher vorteilhaft ein Mikro-Elektro-Mechanisches System.

Vorgeschlagen wird nach dem zuvor Gesagten vorteilhaft ein Schichtaufbau bestehend aus einem vorteilhaft mindestens auf der Einlassseite 61 planaren Heizkörper 60 auf Si-Basis und einer oder mehrerer darunter liegender Kapillarstrukturen 19 mit vorteilhaft unterschiedlicher Porengröße. Die direkt an der Einlassseite 61 des Heizkörpers 60 angeordnete Dochtstruktur 19 verhindert die Bildung von Blasen an der Einlassseite 61 des Heizkörpers 60, da Gasblasen eine weitere Förderwirkung unterbinden und gleichzeitig zu einer (lokalen) Überhitzung des Heizkörpers 60 aufgrund fehlender Kühlung durch nachströmendes Liquid führen.

## Patentansprüche

1. Verdampfereinheit für einen Inhalator, insbesondere für ein elektronisches Zigarettenprodukt, mit einem elektrisch betreibbaren, insbesondere planaren Heizkörper (60), der eine Einlassseite (61) und eine Auslassseite (64) aufweist, und einer Mehrzahl von Mikrokanälen (62), die sich jeweils von der Einlassseite (61) zu der Auslassseite (64) durch den Heizkörper (60) erstrecken, wobei der Heizkörper (60) durch Anlegen einer Heizspannung zum Verdampfen von durch die Mikrokanäle (62) geförderter Flüssigkeit eingerichtet ist, wobei an der Einlassseite (61) des Heizkörpers (60) eine poröse und/oder kapillare Dochtstruktur (19) angeordnet ist, die flächig und kontaktierend an dem Heizkörper (60) anliegt und sämtliche Mikrokanäle (62) an der Einlassseite (61) überdeckt, wobei die Verdampfereinheit ein Substrat (23) mit einer Durchgangsöffnung (25) zum Halten des Heizkörpers (60) und/oder der Dochtstruktur (19) aufweist, **dadurch gekennzeichnet, dass** die mittlere Poren-/Kapillargröße von Poren in der Dochtstruktur (19) im Kontaktbereich zu dem Heizkörper (60) kleiner, vorzugsweise um mindestens eine Größenordnung kleiner ist als der kleinste lichte Durchmesser der Mikrokanäle (62).

2. Verdampfereinheit nach Anspruch 1, **dadurch gekennzeichnet, dass** die mittlere Poren-/Kapillargröße von Poren oder Kapillaren in der Dochtstruktur (19) im Kontaktbereich zu dem Heizkörper (60) kleiner, vorzugsweise um mindestens eine Größenordnung kleiner ist als der kleinste Abstand zweier Mikrokanäle (62) zueinander.

3. Verdampfereinheit nach einem der vorangehenden Ansprüche, **dadurch gekennzeichnet, dass** sich die mittlere Poren-/Kapillargröße in der Dochtstruktur (19) mit abnehmendem Abstand zum Heizkörper (60) insbesondere monoton verändert.

4. Verdampfereinheit nach Anspruch 34, **dadurch gekennzeichnet, dass** sich die mittlere Poren-/Kapillargröße in der Dochtstruktur (19) mit abnehmendem Abstand zum Heizkörper (60) insbesondere monoton verringert.

5. Verdampfereinheit nach einem der Ansprüche 34 oder 4,
**dadurch gekennzeichnet, dass** die Dochtstruktur (19) eine oder mehrere porösen und/oder kapillaren Schichten aufweist, deren Poren oder Kapillaren jeweils eine konstante mittlere Poren-/Kapillargröße aufweisen.

6. Verdampfereinheit nach einem der Ansprüche 34 oder 4,
**dadurch gekennzeichnet, dass** die Dochtstruktur (19) eine poröse und/oder kapillare Schicht mit gradueller Veränderung, insbesondere Abnahme der Poren-/Kapillargröße in Richtung zu dem Heizkörper hin umfasst.

7. Verdampfereinheit nach einem der vorangehenden Ansprüche, **dadurch gekennzeichnet, dass** die Förderrate der Dochtstruktur (19) mindestens so groß ist wie die maximale Verdampfungsrate des Heizkörpers (60).

8. Verdampfereinheit nach einem der vorangehenden Ansprüche, **dadurch gekennzeichnet, dass** die Dochtstruktur aus einem oder mehreren aus der Gruppe der Materialien Baumwolle, Cellulose, Acetat, Glasfasergewebe, Glasfaserkeramik, Sinterkeramik, keramisches Papier, Alumosilikat-Papier, Metallschaum, Metallschwamm, einem anderen hitzebeständigen, porösen und/oder kapillaren Material mit geeigneter Förderrate, oder einem Verbund von zwei oder mehr der vorgenannter Materialien besteht.

9. Verdampfereinheit nach einem der vorangehenden Ansprüche, **dadurch gekennzeichnet, dass** die Dochtstruktur (19) eine Filterschicht (55) insbesondere aus Mikroglasfaser aufweist.

10. Verdampfereinheit nach einem der vorangehenden Ansprüche, **dadurch gekennzeichnet, dass** die Dochtstruktur (19) eine Faserpapier-Schicht (56) aufweist.

11. Verdampfereinheit nach einem der vorangehenden Ansprüche, **dadurch gekennzeichnet, dass** die Dochtstruktur (19) eine Keramikdocht-Schicht (57) aufweist.

12. Verdampfereinheit nach einem der vorangehenden Ansprüche, **dadurch gekennzeichnet, dass** die Dochtstruktur (19) eine Öllampendocht-Schicht (58) aufweist.

13. Verdampfereinheit nach einem der vorangehenden Ansprüche, **dadurch gekennzeichnet, dass** die Verdampfereinheit ein insbesondere als Leiterplatte ausgebildetes Haltelement (26) mit einer Durchgangsöffnung (27) zum Halten der Dochtstruktur (19) aufweist.

14. Verdampfereinheit nach einem der vorangehenden Ansprüche, **dadurch gekennzeichnet, dass** Frequenz und/oder Tastgrad einer Heizspannung Uh für den Heizkörper (60) an die Eigenschwingung und/oder Eigenfrequenz von Schwingungen von in den Mikrokanälen (62) entstehenden Gasblasen angepasst ist.

15. Verdampfereinheit nach Anspruch 14, **dadurch gekennzeichnet, dass** Frequenz und/oder Tastgrad der Heizspannung Uh je nach Mischungsverhältnis der zu verdampfenden Flüssigkeit optimal gewählt ist.

16. Verdampfereinheit nach Anspruch 14 oder 15, **dadurch gekennzeichnet, dass** die Frequenz der Heizspannung Uh im Bereich zwischen 20 Hz und 200 Hz liegt.

17. Verdampfereinheit nach einem der vorangehenden Ansprüche, **dadurch gekennzeichnet, dass** der durch eine Heizspannung Uh für den Heizkörper (60) erzeugte maximale Heizstrom nicht mehr als 7 A beträgt.

18. Verdampfereinheit nach einem der vorangehenden Ansprüche, **dadurch gekennzeichnet, dass** die Verdampfereinheit (20) mindestens ein eine Vorspannung erzeugendes Klemmelement (37) aufweist, das zur Klemmung des Heizkörpers (60) auf den Träger (23) angeordnet und eingerichtet ist.

19. Verdampfereinheit nach Anspruch 18, **dadurch gekennzeichnet, dass** mindestens zwei Klemmelemente (37) auf entgegengesetzten Seiten des Heizkörpers (60) vorgesehen sind.

20. Verdampfereinheit nach Anspruch 18 oder 19, **dadurch gekennzeichnet, dass** das mindestens eine Klemmelement (37) einen den Heizkörper (60) linienförmig kontaktierenden Klemmbügel (38) aufweist.

21. Verdampfereinheit nach einem der Ansprüche 18 bis 20, **dadurch gekennzeichnet, dass** das mindestens eine Klemmelement (37) den Heizkörper (60) seitlich parallel zur Auslassseite und/oder senkrecht auf die Auslassseite (64) und/oder in einer Ecknut des Trägers (23) klemmt.

22. Verdampfereinheit nach einem der Ansprüche 18 bis 21, **dadurch gekennzeichnet, dass** das mindestens eine Klemmelement (37) als Elektrode zur elektrischen Kontaktierung und Versorgung des Heizkörpers (60) dient.

23. Verdampfereinheit nach Anspruch 22, **dadurch gekennzeichnet, dass** mindestens ein sich durch eine Bohrung (45) des Trägers (23) erstreckender Leiter (12) zur Kontaktierung des Klemmelements (37) vorgesehen ist.

24. Verdampfereinheit nach Anspruch 23, **dadurch gekennzeichnet, dass** der mindestens eine Leiter (12) eine Leiterplatte (26) kontaktiert, die auf der von dem Heizkörper (60) abgewandten Seite des Trägers (23) beabstandet angeordnet ist.

25. Verdampfereinheit nach einem der vorangehenden Ansprüche, **dadurch gekennzeichnet, dass** der Träger (23) als Leiterplatte ausgebildet ist.

## Claims

1. Evaporator unit for an inhaler, in particular for an electronic cigarette product, comprising an electrically operable, in particular planar, heating body (60) which has an inlet side (61) and an outlet side (64), and a plurality of microchannels (62) which each extend from the inlet side (61) to the outlet side (64) through the heating body (60), the heating body (60) being designed to evaporate, by applying a heating voltage, liquid transferred through the microchannels (62), wherein a porous and/or capillary wick structure (19) is arranged on the inlet side (61) of the heating body (60), which structure rests flat against and in contact with the heating body (60) and covers all of the microchannels (62) on the inlet side (61), wherein the evaporator unit has a substrate (23) comprising a through-opening (25) for holding the heating body (60) and/or the wick structure (19), **characterised in that** the average pore/capillary size of pores in the wick structure (19) in the contact region with the heating body (60) is smaller, preferably at least one order of magnitude smaller, than the smallest inside diameter of the microchannels (62).

2. Evaporator unit according to claim 1, **characterised in that** the average pore/capillary size of pores or capillaries in the wick structure (19) in the contact region with the heating body (60) is smaller, preferably at least one order of magnitude smaller, than the smallest distance of two microchannels (62) from one another.

3. Evaporator unit according to any of the preceding claims,
**characterised in that** the average pore/capillary size in the wick structure (19) changes in particular monotonically as the distance from the heating body (60) decreases.

4. Evaporator unit according to claim 3, **characterised in that** the average pore/capillary size in the wick structure (19) reduces in particular monotonically as the distance from the heating body (60) decreases.

5. Evaporator unit according to either claim 3 or claim 4, **characterised in that** the wick structure (19) has one or more porous and/or capillary layers, the pores or capillaries of which each have a constant average pore/capillary size.

6. Evaporator unit according to either claim 3 or claim 4, **characterised in that** the wick structure (19) comprises a porous and/or capillary layer having a gradual change, in particular a decrease in the pore/capillary size in the direction towards the heating body.

7. Evaporator unit according to any of the preceding claims,
**characterised in that** the transfer rate of the wick structure (19) is at least as great as the maximum evaporation rate of the heating body (60).

8. Evaporator unit according to any of the preceding claims,
**characterised in that** the wick structure consists of one or more from the group of the materials cotton, cellulose, acetate, glass fibre fabric, glass fibre ceramic, sintered ceramic, ceramic paper, aluminosilicate paper, metal foam, metal sponge, another heat-resistant, porous and/or capillary material having a suitable transfer rate, or a combination of two or more of the aforementioned materials.

9. Evaporator unit according to any of the preceding claims,
**characterised in that** the wick structure (19) has a filter layer (55), in particular made of micro-glass fibre.

10. Evaporator unit according to any of the preceding claims,
**characterised in that** the wick structure (19) has a fibre paper layer (56).

11. Evaporator unit according to any of the preceding claims,
**characterised in that** the wick structure (19) has a ceramic wick layer (57).

12. Evaporator unit according to any of the preceding claims,
**characterised in that** the wick structure (19) has an oil lamp wick layer (58).

13. Evaporator unit according to any of the preceding claims,
**characterised in that** the evaporator unit has a holding element (26), in particular in the form of a circuit board, comprising a through-opening (27) for holding the wick structure (19).

14. Evaporator unit according to any of the preceding claims,
**characterised in that** the frequency and/or duty cycle of a heating voltage Uh for the heating body (60) is adapted to the natural vibration and/or natural frequency of vibrations of gas bubbles formed in the microchannels (62).

15. Evaporator unit according to claim 14, **characterised in that** the frequency and/or duty cycle of the heating voltage Uh is optimally selected depending on the mixing ratio of the liquid to be evaporated.

16. Evaporator unit according to either claim 14 or 15, **characterised in that** the frequency of the heating voltage Uh is in the range between 20 Hz and 200 Hz.

17. Evaporator unit according to any of the preceding claims,
**characterised in that** the maximum heating current generated by a heating voltage Uh for the heating body (60) is not more than 7 A.

18. Evaporator unit according to any of the preceding claims,
**characterised in that** the evaporator unit (20) has at least one clamping element (37) which generates a preload and is arranged and designed to clamp the heating body (60) onto the support (23).

19. Evaporator unit according to claim 18, **characterised in that** at least two clamping elements (37) are provided on opposite sides of the heating body (60).

20. Evaporator unit according to either claim 18 or claim 19, **characterised in that** the at least one clamping element (37) has a clamping bracket (38) which makes linear contact with the heating body (60).

21. Evaporator unit according to any of claims 18 to 20, **characterised in that** the at least one clamping element (37) clamps the heating body (60) laterally to and in parallel with the outlet side and/or vertically onto the outlet side (64) and/or in a corner groove of the support (23).

22. Evaporator unit according to any of claims 18 to 21, **characterised in that** the at least one clamping element (37) is used as an electrode for electrically contacting and supplying power to the heating body (60).

23. Evaporator unit according to claim 22, **characterised in that** at least one conductor (12) extending through a hole (45) in the support (23) is provided for contacting the clamping element (37).

24. Evaporator unit according to claim 23, **characterised in that** the at least one conductor (12) contacts a circuit board (26) which is arranged on the side of the support (23) and spaced apart from the support (23) facing away from the heating body (60).

25. Evaporator unit according to any of the preceding claims,
**characterised in that** the support (23) is in the form of a circuit board.

## Revendications

1. Unité de vaporisation pour un inhalateur, en particulier pour un produit sous forme de cigarette électronique, comportant un corps chauffant (60), en particulier plan, pouvant être actionné électriquement, qui présente un côté entrée (61) et un côté sortie (64), et une pluralité de micro-conduits (62) qui s'étendent chacun du côté entrée (61) au côté sortie (64) à travers le corps chauffant (60), le corps chauffant (60) étant conçu pour vaporiser un liquide transporté à travers les micro-conduits (62) par application d'une tension de chauffage, une structure de mèche (19) poreuse et/ou capillaire étant disposée sur le côté entrée (61) du corps chauffant (60), laquelle s'applique à plat et en contact avec le corps chauffant (60) et recouvre tous les micro-conduits (62) sur le côté entrée (61), l'unité de vaporisation présentant un substrat (23) avec une ouverture de passage (25) pour maintenir le corps chauffant (60) et/ou la structure de mèche (19), **caractérisée en ce que** la taille de pores/capillaires moyenne des pores dans la structure de mèche (19) dans la zone de contact avec le corps chauffant (60) est inférieure, de préférence inférieure d'au moins un ordre de grandeur, au plus petit diamètre intérieur des micro-conduits (62).

2. Unité de vaporisation selon la revendication 1, **caractérisée en ce que** la taille de pores/capillaires moyenne des pores ou capillaires dans la structure de mèche (19) dans la zone de contact avec le corps chauffant (60) est inférieure, de préférence inférieure d'au moins un ordre de grandeur, à la plus petite distance entre deux micro-conduits (62).

3. Unité de vaporisation selon l'une des revendications précédentes, **caractérisée en ce que** la taille de pores/capillaires moyenne dans la structure de mèche (19) varie, en particulier de manière monotone, à mesure que la distance au corps chauffant (60) diminue.

4. Unité de vaporisation selon la revendication 3, **caractérisée en ce que** la taille de pores/capillaires moyenne dans la structure de mèche (19) s'amoindrit, en particulier de manière monotone, à mesure que la distance au corps chauffant (60) diminue.

5. Unité de vaporisation selon l'une des revendications 3 ou 4, **caractérisée en ce que** la structure de mèche (19) présente une ou plusieurs couches poreuses et/ou capillaires dont les pores ou capillaires présentent chaque fois une taille de pores/capillaires moyenne constante.

6. Unité de vaporisation selon l'une des revendications 3 ou 4, **caractérisée en ce que** la structure de mèche (19) comprend une couche poreuse et/ou capillaire avec une variation graduelle, en particulier une diminution de la taille de pores/capillaires en direction du corps chauffant.

7. Unité de vaporisation selon l'une des revendications précédentes, **caractérisée en ce que** le débit de transport de la structure de mèche (19) est au moins aussi grand que le débit de vaporisation maximal du corps chauffant (60).

8. Unité de vaporisation selon l'une des revendications précédentes, **caractérisée en ce que** la structure de mèche est constituée d'un ou de plusieurs matériaux choisis dans le groupe constitué par le coton, la cellulose, l'acétate, le tissu de fibres de verre, la céramique à fibres de verre, la céramique frittée, le papier céramique, le papier aluminosilicate, la mousse métallique, l'éponge métallique, un autre matériau résistant à la chaleur, poreux et/ou capillaire avec un débit de transport approprié, ou un composite de deux ou plusieurs des matériaux précités.

9. Unité de vaporisation selon l'une des revendications précédentes, **caractérisée en ce que** la structure de mèche (19) présente une couche filtrante (55), en particulier en microfibres de verre.

10. Unité de vaporisation selon l'une des revendications précédentes, **caractérisée en ce que** la structure de mèche (19) présente une couche de papier fibreux (56).

11. Unité de vaporisation selon l'une des revendications précédentes, **caractérisée en ce que** la structure de mèche (19) présente une couche de mèche céramique (57).

12. Unité de vaporisation selon l'une des revendications précédentes, **caractérisée en ce que** la structure de mèche (19) présente une couche de mèche de lampe à huile (58).

13. Unité de vaporisation selon l'une des revendications précédentes, **caractérisée en ce que** l'unité de vaporisation présente un élément de maintien (26), réalisé en particulier sous la forme d'une carte de circuit imprimé, avec une ouverture de passage (27) pour maintenir la structure de mèche (19).

14. Unité de vaporisation selon l'une des revendications précédentes, **caractérisée en ce que** la fréquence et/ou le rapport cyclique d'une tension de chauffage Uh pour le corps chauffant (60) est adapté(e) à l'oscillation propre et/ou à la fréquence propre d'oscillations de bulles de gaz se formant dans les micro-conduits (62).

15. Unité de vaporisation selon la revendication 14, **caractérisée en ce que** la fréquence et/ou le rapport cyclique de la tension de chauffage Uh est choisi(e) de manière optimale en fonction du rapport de mélange du liquide à vaporiser.

16. Unité de vaporisation selon la revendication 14 ou 15, **caractérisée en ce que** la fréquence de la tension de chauffage Uh se situe dans la plage comprise entre 20 Hz et 200 Hz.

17. Unité de vaporisation selon l'une des revendications précédentes, **caractérisée en ce que** le courant de chauffage maximal généré par une tension de chauffage Uh pour le corps chauffant (60) n'est pas supérieur à 7 A.

18. Unité de vaporisation selon l'une des revendications précédentes, **caractérisée en ce que** l'unité de vaporisation (20) présente au moins un élément de serrage (37) générant une précontrainte, qui est disposé et conçu pour serrer le corps chauffant (60) sur le support (23).

19. Unité de vaporisation selon la revendication 18, **caractérisée en ce qu'**au moins deux éléments de serrage (37) sont prévus sur des côtés opposés du corps chauffant (60).

20. Unité de vaporisation selon la revendication 18 ou 19, **caractérisée en ce que** ledit au moins un élément de serrage (37) présente un étrier de serrage (38) en contact linéaire avec le corps chauffant (60).

21. Unité de vaporisation selon l'une des revendications 18 à 20, **caractérisée en ce que** ledit au moins un élément de serrage (37) serre le corps chauffant (60) latéralement parallèlement au côté sortie et/ou perpendiculairement au côté sortie (64) et/ou dans une rainure d'angle du support (23).

22. Unité de vaporisation selon l'une des revendications 18 à 21, **caractérisée en ce que** ledit au moins un élément de serrage (37) sert d'électrode pour la mise en contact électrique et l'alimentation du corps chauffant (60).

23. Unité de vaporisation selon la revendication 22, **caractérisée en ce qu'**au moins un conducteur (12) s'étendant à travers un alésage (45) du support (23) est prévu pour la mise en contact de l'élément de serrage (37).

24. Unité de vaporisation selon la revendication 23, **caractérisée en ce que** ledit au moins un conducteur (12) est en contact avec une carte de circuit imprimé (26) qui est disposée à distance sur le côté du support (23) opposé au corps chauffant (60).

25. Unité de vaporisation selon l'une des revendications précédentes, **caractérisée en ce que** le support (23) est réalisé sous la forme d'une carte de circuit imprimé.
